# EUROPEAN PATENT SPECIFICATION

(11) **EP 1 318 183 B1**
(45) Date of publication and mention of the grant of the patent: **18.05.2005**
(21) Application number: 02025129.4
(22) Date of filing: 09.11.2002
(51) Int. Cl.: C09K 11/06

(54) **Oligomers and Polymers comprising a 2,6-azulene group and their use as charge transport materials**
Oligomere und Polymere enthaltend eine 2,6-azulene Gruppe und ihre Verwendung als Ladungstransport Materialien
Oligomeres et Polymeres comprenant un group 2,6-azulene et leurs utilisation comme matériaux de transport de charges

(30) Priority: 10.12.2001 EP 01129216
(43) Date of publication of application: 11.06.2003
(73) Proprietor: MERCK PATENT GmbH, 64293 Darmstadt (DE)
(72) Inventor: Farrand, Louise, Blandford Forum, Dorset, DT11 9ED (GB); Findlater, Michael, Glasgow, G69 7QG (GB); Giles, Mark, Southampton, SO15 2LE (GB); Heeney, Martin, Southampton, SO14 6TQ (GB); Tierney, Steven, Southampton SO15 7QW (GB); Thompson, Marcus, Fordingbridge, Hampshire, SP6 1RR (GB); Shkunov, Maxim, Southampton, SO16 6SX (GB); Sparrowe, David, Bournemouth, Dorset, BH6 5EJ (GB); McCulloch, Iain, Hampshire, SO20 6PE (GB)

(56) References cited:
- US-A- 5 728 867
- D. BALSCHUKAT; E.V.DEMLOW: "Neuartige 2,6-disubstituierte Azulene" CHEMISCHE BERICHTE, vol. 119, no. 7, 1986, pages 2272-2288, XP002236201
- SAITOH MASAKI ET AL: "Voltammetric behavior and electronic molecular structures of several azulenylketones" J ELECTROANAL CHEM;JOURNAL OF ELECTROANALYTICAL CHEMISTRY NOV 21 1996, vol. 418, no. 1-2, 21 November 1996 (1996-11-21), pages 139-145, XP002236202

## Description

### Field of Invention

The invention relates to new conjugated oligo- and polyazulenes. The invention further relates to their use as semiconductors or charge transport materials in optical, electrooptical or electronic devices including field effect transistors, electroluminescent, photovoltaic and sensor devices. The invention further relates to field effect transistors and semi-conducting components comprising the new oligo- and polyazulenes.

### Background and Prior Art

Organic materials have recently shown promise as the active layer in organic based thin film transistors and organic field effect transistors [see H. E. Katz, Z. Bao and S. L. Gilat, *Acc. Chem. Res.,* 2001, **34,** 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semi-conducting material and the current on/off ratio, so the ideal semi-conductor should have a low conductivity in the off state, combined with a high charge carrier mobility (> 1 x 10⁻³ cm² V⁻¹ s⁻¹). In addition, it is important that the semi-conducting material is relatively stable to oxidation i.e. it has a high ionisation potential, as oxidation leads to reduced device performance.

A known compound which has been shown to be an effective p-type semiconductor for OFETs is pentacene [see S. F. Nelson, Y. Y. Lin, D. J. Gundlach and T. N. Jackson, *Appl. Phys. Lett*., 1998, **72,** 1854]. When deposited as a thin film by vacuum deposition, it was shown to have carrier mobilities in excess of 1 cm² V⁻¹ s⁻¹ with very high current on/off ratios greater than 10⁶. However, vacuum deposition is an expensive processing technique that is unsuitable for the fabrication of large-area films.

It is the aim of the present invention to provide new materials for use as semiconductors or charge transport materials, which are easy to synthesise, have high charge mobility, good processability and improved oxidative stability. Other aims of the invention are immediately evident to those skilled in the art from the following description.

The inventors have found that these aims can be achieved by providing new oligomers and polymers based on azulene.

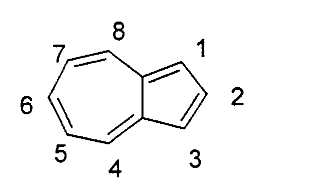

Azulene is a non-benzenoid aromatic hydrocarbon which is planar and thermodynamically stable. Polymerisation at the 2- or 6-position results in a linear structure. As a result, polyazulenes pack closely, thus exhibiting a higher degree of order that leads to particularly high charge carrier mobility. Furthermore, by adding alkyl chains and other substituent groups to the azulene core, the azulenes can be made more soluble thus being suitable for spin coating or solution coating techniques, rather than vacuum deposition, to prepare thin films for use e.g. in electronic devices such as transistors.

1,3-Polyazulenes (**A**) have been prepared electrochemically, as reported by K. Iwasaki et al, *Synth. Metals*, 1995, 69, 543 and Y-B. Shim et al, *J. Electrochem. Soc.,* 1997, 144, 3027 and M. Porsch et al in *Adv. Mater.,* 1997, 9, 635, and by stirring in strong acid, as reported by N. Kihara et al, *J. Amer. Chem. Soc.,* 1997, 30, 6385. Azulene appended cellulose has also been reported [see F. X. Redl et al, *Macromol. Chem. Phys.,* 2000, 201, 2091 ]

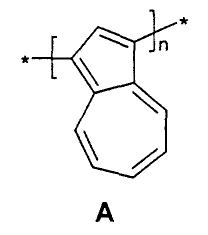

Copolymers of azulene have also been reported. DE 34 25 511, DE 3929383 and DE 39 38 094 disclose a copolymerisate of pyrrole and azulene obtained by electrochemical polymerisation in the presence of sulfonic acid and a conducting salt. DE 44 45 619 discloses a copolymer with azulene and phenylene units linked by phenylmethylene groups.

However, polyazulenes polymerised at the 2- and 6-position according to the present invention have not been reported.

Another aspect of the present inventions relates to advantageous uses of the oligo- and polyazulenes, including their oxidatively or reductively doped forms, according to the invention.

### Definition of Terms

The term 'film' includes self-supporting, i.e. free-standing, films that show more or less pronounced mechanical stability and flexibility, as well as coatings or layers on a supporting substrate or between two substrates.

### Summary of the Invention

One object of the invention are oligo- and polymers comprising at least two azulene-2,6-diyl groups.

Another object of the invention is the use of mono-, oligo- and polyazulenes according to the invention as semiconductors or charge transport materials, in particular in optical, electrooptical or electronic devices, like for example components of integrated circuitry, field effect transistors (FET) for example as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors and for electrophotographic applications like electrophotographic recording.

Another object of the invention is a field effect transistor, for example as a component of integrated circuitry, as a thin film transistor in flat panel display applications, or in a Radio Frequency Identification (RFID) tag, comprising one or more oligo- or polyazulenes according to the invention.

Another object of the invention is a semi-conducting component, for example in OLED applications like electroluminescent displays or backlights of e.g. liquid crystal displays, in photovoltaic or sensor devices, as electrode materials in batteries, as photoconductors and for electrophotographic applications, comprising one or more oligo- or polymers according to the invention.

Another object of the invention is a security marking or device comprising an RFID or ID tag or a FET according to the invention.

### Detailed Description of the Invention

The oligo- and polyazulenes according to the present invention are especially useful as charge transport semiconductors in that they have high carrier mobilities. Particularly preferred are oligo- and polyazulenes wherein the azulene group is substituted by one or more alkyl or fluoroalkyl groups. The introduction of alkyl side chains to the azulene group and attached rings improves the solubility and therefore the solution processibility of the inventive materials.

Especially preferred are oligo- and polymers comprising identical or different recurring units of formula I

-[(A)ₐ-(B)_{b}-(C)_{c}]- I

wherein
- A and C: are independently of each other and independently in each occurrence -CX¹=CX²-, -C≡C-, or optionally substituted arylene or heteroarylene, or have one of the meanings of B,
- X¹ and X²: are independently of each other H, F, Cl or CN,
- B: is independently in each occurrence azulene-2,6-diyl, [2,6']-bisazulene-6,2'-diyl, [2,2']-bisazulene-6,6'-diyl, [6,6']-bisazulene-2,2'-diyl, all of which are substituted or unsubstituted, or a mirror image of one of these groups, and
- a, b and c: are independently of each other 0, 1 or 2, with a + b + c > 0,
with the proviso that the oligo- and polyazulenes comprise at least two azulene-2,6-diyl groups or at least one [2,6']-bisazulene-6,2'-diyl, [2,2']-bisazulene-6,6'-diyl or [6,6']-bisazulene-2,2'-diyl group.

In the oligo- and polymers of the present invention the recurring units (A)ₐ-(B)_{b}-(C)_{c} in case of multiple occurrence can be selected of formula I independently of each other, so that an oligo- or polymer may comprise identical or different recurring units (A)ₐ-(B)_{b}-(C)_{c}. The oligo- and polymers thus include homopolymers and copolymers like for example
- statistically random copolymers, for example with a monomer sequence such as -A-B-C-C-B-A-B-,
- alternating copolymers, for example with a monomer sequence such as -A-B-C-A-B-C-, and
- block copolymers, for example with a monomer sequence such as -A-A-B-B-B-B-C-C-C-,
wherein the groups A and C preferably form a conjugated system together with the group B.

Further preferred are oligo- and polymers comprising recurring units (A)ₐ-(B)_{b}-(C)_{c}, wherein a = c = 0 and b = 1, very preferably consisting exclusively of such recurring units.

Further preferred are oligo- and polymers comprising recurring units (A)ₐ-(B)_{b}-(C)_{c}, wherein b = c = 1 and a = 0, very preferably consisting exclusively of such recurring units.

Further preferred are oligo- and polymers comprising recurring units (A)ₐ-(B)_{b}-(C)_{c}, wherein a = b = c = 1, very preferably consisting exclusively of such recurring units.

Especially preferred are oligo- and polymers of formula I1

R⁶-[(A)ₐ-(B)_{b}-(C)_{c}]ₙ- R² I1

wherein A, C, a, b and c are as defined in formula I,
- B: is independently in each occurrence a group selected of formulae Ila to IId or their mirror images

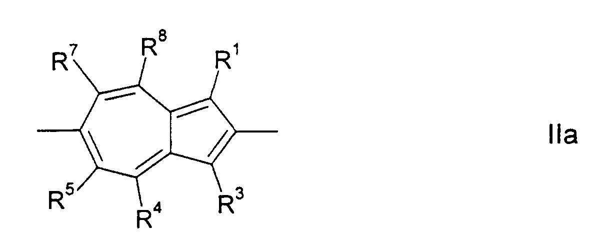

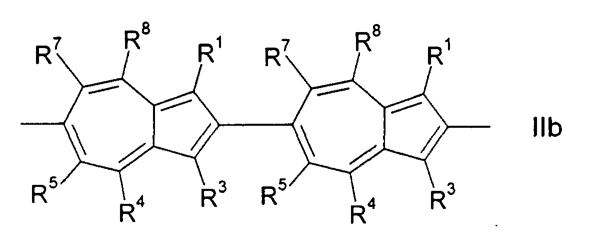

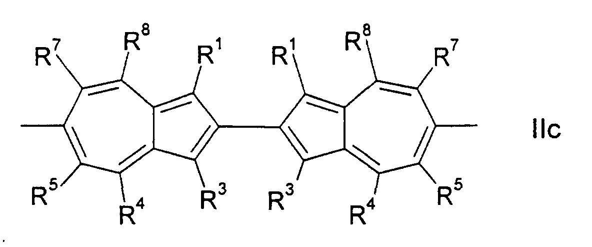

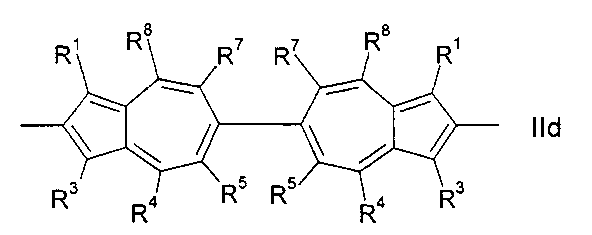

- R¹ to R⁸: are independently of each other H, halogen or straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl,
- R⁰ and R⁰⁰: are independently of each other H or alkyl with 1 to 12 C-atoms,
- n: is an integer from 1 to 5000,
wherein the recurring units [(A)ₐ-(B)_{b}-(C)_{c}] can be identical or different.

Especially preferred are oligo- and polymers of formula I1 wherein
- n is an integer from 2 to 5000, in particular from 20 to 1000,
- n is an integer from 2 to 5,
- the molecular weight is from 5000 to 100000,
- R¹, R³, R⁴, R⁵, R⁷ and R⁸ are selected from H, C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, and optionally substituted aryl or heteroaryl,
- R² and R⁶ are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, and optionally substituted aryl or heteroaryl,
- A and C are optionally substituted arylene or heteroarylene,
- A and C are -CX¹=CX²- or -C≡C-,
- in at least one monomer unit (A)ₐ-(B)_{b}-(C)_{c} a, b and c are 1, and one of A and C is arylene or heteroarylene and the other is -CX¹=CX²-or -C≡C-,
- n>1.

Especially preferred are oligo- and polymers of the following formulae 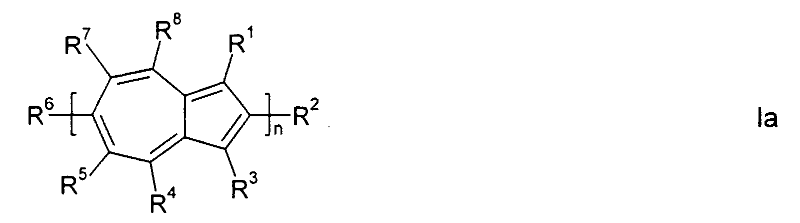 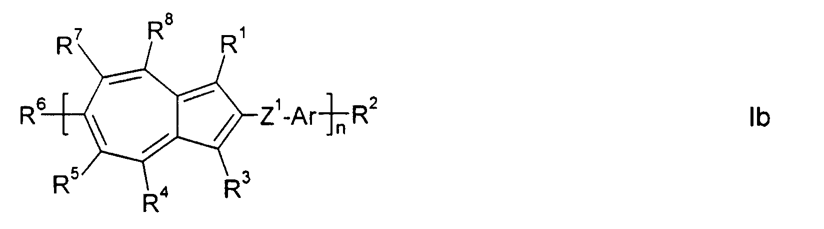 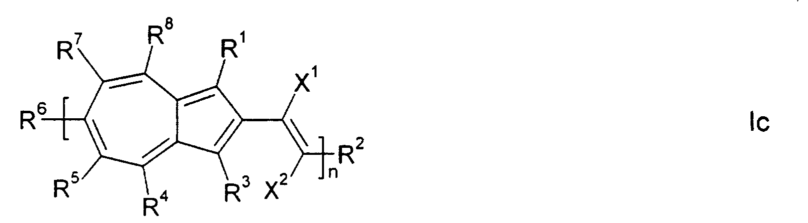 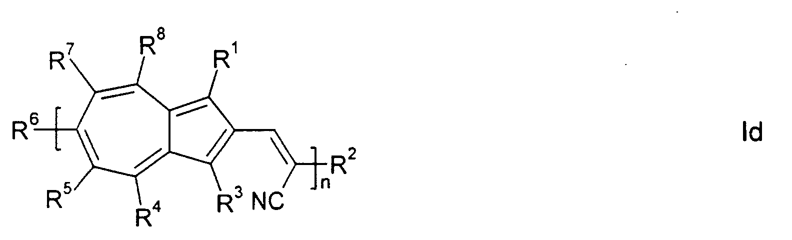 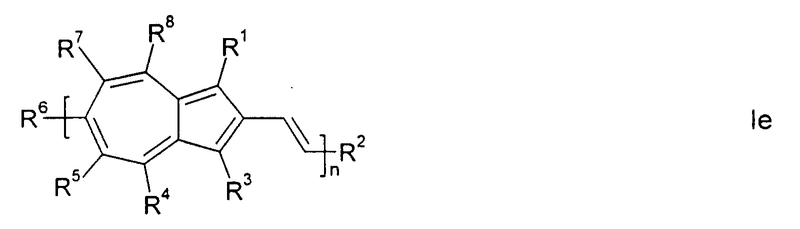 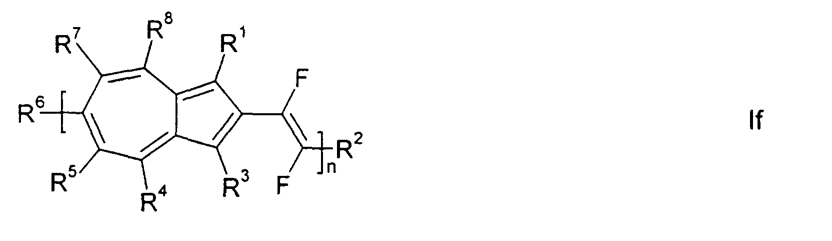 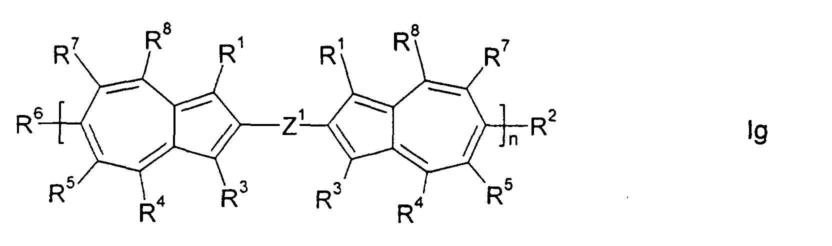 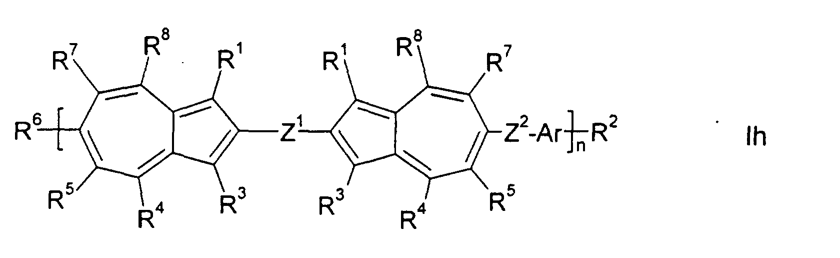 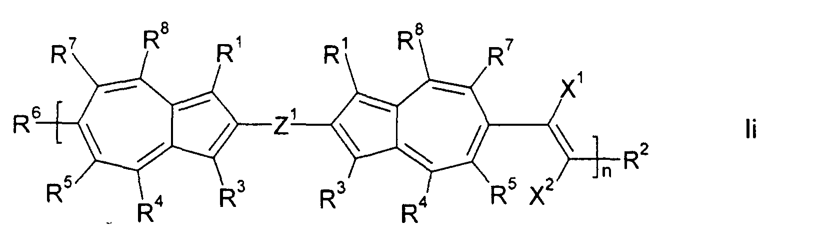 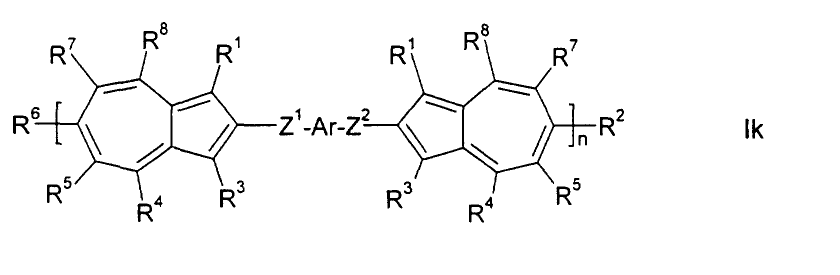 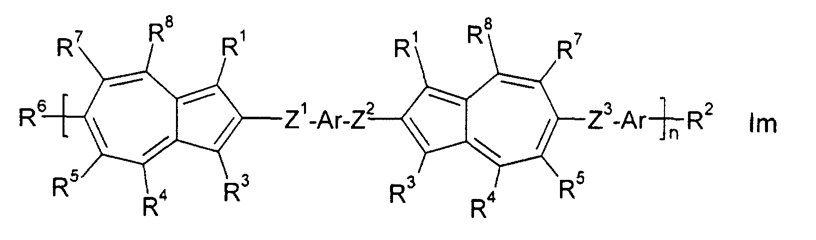 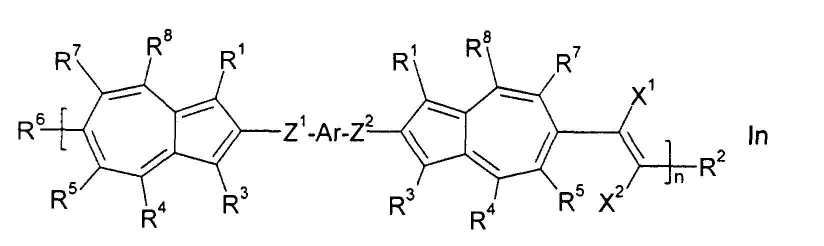 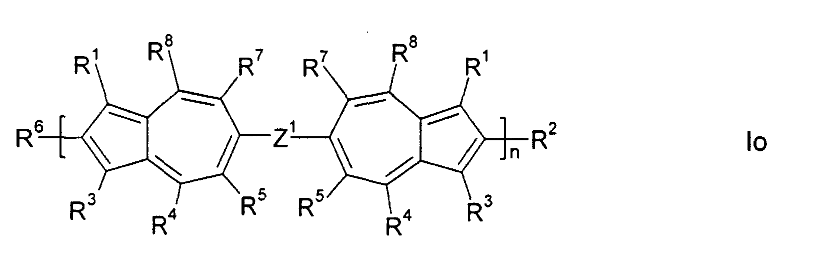 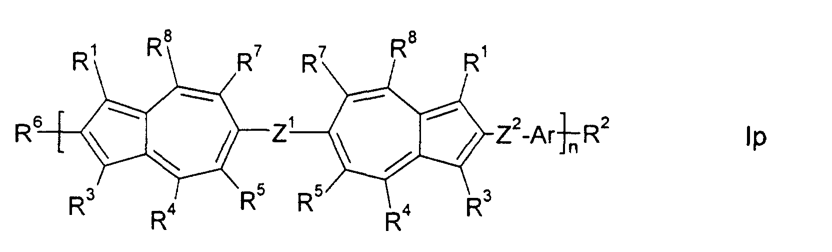 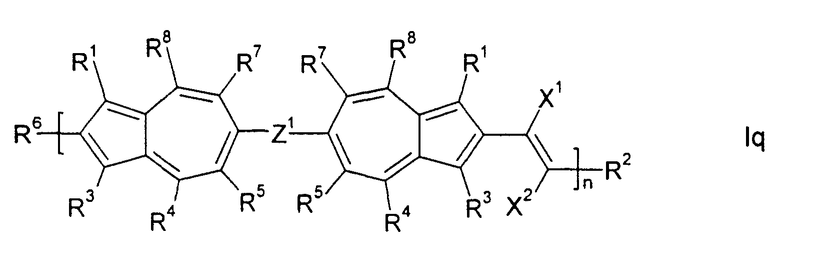 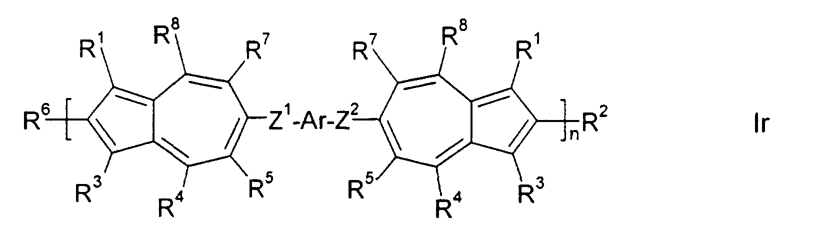 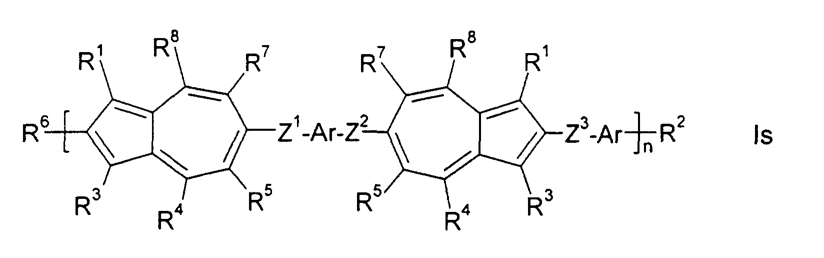 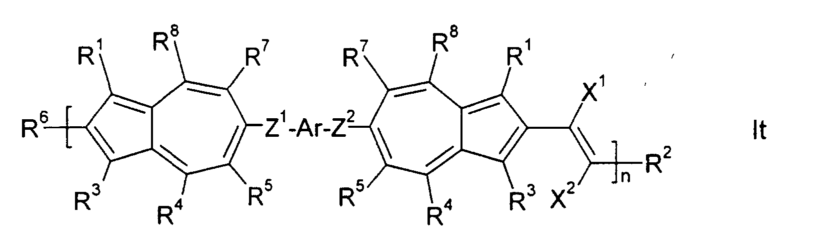 wherein R¹ to R⁸ and n have the meanings given in formula I1,
- Ar: is (ar)ₘ, with ar being arylene or heteroarylene and m being 1, 2 or 3,
- Z¹, Z² and Z³: are independently of each other -CX¹=CX²-, C≡C- or a single bond, and
- X¹ and X²: have the meanings given in formula I.

In these preferred formulae, R¹, R³, R⁴, R⁵, R⁷ and R⁸ are very preferably F or alkyl with 1 to 16 C-atoms that is optionally fluorinated, R² and R⁶ are very preferably H, halogen or alkyl with 1-16 C atoms that is optionally fluorinated, ar is very preferably 1,4-phenylene, alkoxyphenylene, alkylfluorene, thiophene-2,5-diyl, thienothiophene-2,5-diyl or dithienothiophene-2,6-diyl, m is preferably 1, n is preferably an integer from 2 to 5000, in particular from 20 to 1000.

In the formulae shown above and below, aryl and heteroaryl preferably denote a mono-, bi- or tricyclic aromatic or heteroaromatic group with up to 25 C atoms that optionally comprises fused rings and is optionally substituted with one or more groups selected from H, halogen and straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another.

Especially preferred aryl and heteroaryl groups are phenyl in which, in addition, one or more CH groups are optionally replaced by N, naphthalene, thiophene, thienothiophene, dithienothiophene, alkyl fluorene and oxazole, all of which are unsubstituted, mono- or polysubstituted with L, wherein L is halogen or an alkyl, alkoxy, alkylcarbonyl or alkoxycarbonyl group with 1 to 12 C atoms, wherein one or more H atoms are optionally replaced by F or Cl.

Arylene and heteroarylene preferably denote a bivalent mono-, bi- or tricyclic aromatic or heteroaromatic group with up to 25 C atoms that optionally comprises fused rings and is optionally substituted with one or more groups selected from H, halogen and straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another.

Especially preferred arylene and heteroarylene groups are 1,4-phenylene in which, in addition, one or more CH groups are optionally replaced by N, naphthalene-2,6-diyl, thiophene-2,5-diyl, thienothiophene-2,5-diyl, dithienothiophene-2,6-diyl, alkyl fluorene and oxazole, all of which are unsubstituted, mono- or polysubstituted with L as defined above.

Further preferred aryl and heteroaryl groups include five-membered heterocyclics like oxazole or isoxazole, N-substituted imidazole or pyrazole, thiazole or isothiazole, oxadiazole, N-substituted triazole, six-membered heterocyclics like pyridine, pyridazine, pyrimidine, pyrazine, triazine and tetrazine, heterocyclics with fused rings like benzoxazole, benzothiazole, benzimidazole, quinoline, isoquinoline, cinnoline, quinazoline, quinoxaline, phthalazine, benzothiadiazole, benzotriazole, benzotriazine, phenazine, phenanthridine, acridine, or condensed polycyclics like acenaphthene, phenanthrene, anthracene, fluoranthene, pyrene, perylene, rubrene, chrysene, naphthacene, coronene or triphenylene, all of which are unsubstituted, mono- or polysubstituted with L as defined above.

CX¹=CX² is preferably -CH=CH-, -CH=CF-, -CF=CH-, -CF=CF-, -CH=C(CN)- or -C(CN)=CH-.

If in the formulae shown above and below, one of R¹ to R⁸ is an alkyl or alkoxy radical, i.e. where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

"Fluorinated alkyl" or "fluoroalkyl" is mono-, poly- or perfluorinated alkyl, preferably CᵢF₂ᵢ₊₁, wherein i is an integer from 1 to 15, in particular CF₃, C₂F₅, C₃F₇, C₄F₉, C₅F₁₁, C₆F₁₃, C₇F₁₅ or C₈F₁₇, very preferably C₆F₁₃.

Halogen is preferably F or Cl.

The oligo- and polyazulenes of the present invention can be synthesized according to or in analogy to known methods. Some preferred methods are described below.

As shown in Scheme 1, Ullmann -type coupling (see T. Morita and K. Takase, *Bull. Chem. Soc. Jpn.,* 1982, 55, 1144-1152) gives biazulene (1) followed by bromination (see T. Nozoe, T. Asao and M. Oda in *Bull. Chem. Soc. Jpn.,* 1974, 47, 681) gives compound (2) and decarboxylation (see D. Balschukat and E. V. Dehmlow, *Chem. Ber*., 1986, 119, 2272) gives the 2, 2'-biazulene (3). Polymerisation can proceed via conventional routes: Ni(cod)₂ and triphenylphosphine (Yamamoto coupling) to yield (5). Alternatively, (3) is converted to the mono-Grignard and polymerised using Ni(dppp)Cl₂ to yield (5). Other coupling routes are Stille coupling (see D. Milstein and J. K. Stille, *J. Am. Chem. Soc.* , 1979, 101, 4992), Rieke coupling (see T.-A. Chen and R. D. Rieke, *J. Am. Chem. Soc.,* 1992, 114, 10087), and Suzuki coupling (see N. Miyaura, T. Yanagi and A. Suzuki, *Synth. Commun.,* 1981, 11, 513). 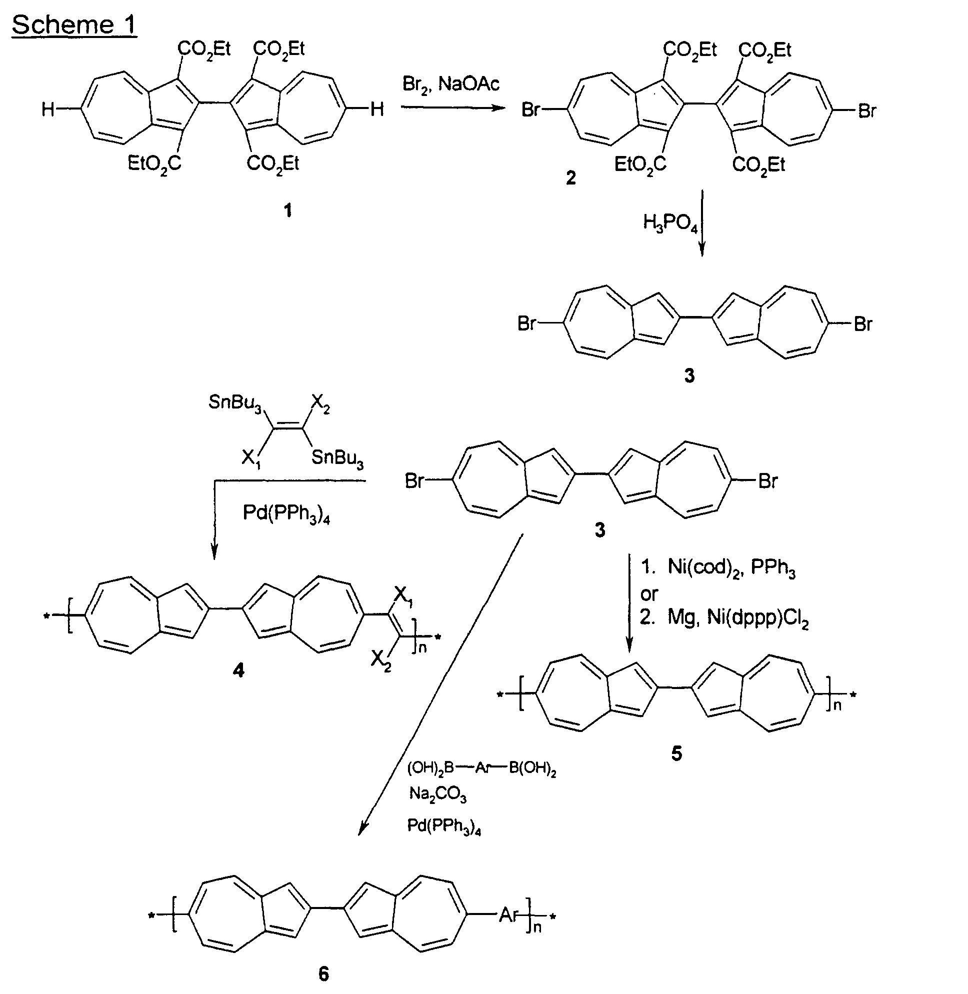 wherein Ar, X¹, X² and n have the meanings given in formula I and I1.

As shown in Scheme 2, **(3)** can be cross-coupled under Sonogashira conditions with 4-chlorophenylacetylene to give the 2, 2' biazulene **(8)**, which can be polymerised under typical and known conditions to give for example highly conjugated polymer **(9)**.

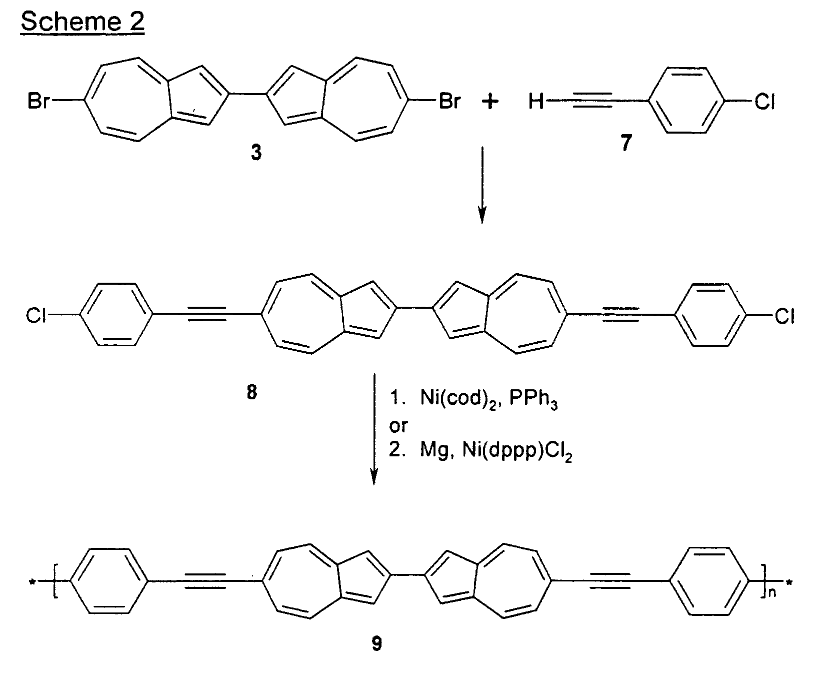

As depicted in scheme 3, compound **(10)** is prepared by bromination of 2-amino-1,3-azulene diethylcarboxylate (see T. Nozoe, S. Seto and S. Matsumara, *Bull. Chem. Soc. Jpn.,* 1962, **35,** 1990). (**11**) is the cross-coupled product of (**10**) with protected acetylene. The amine is converted to the chloro compound **(13)**, decarboxylated with acid and polymerised to give (**15**).

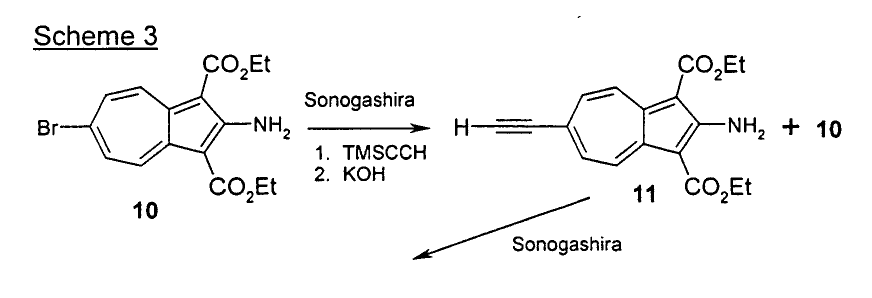

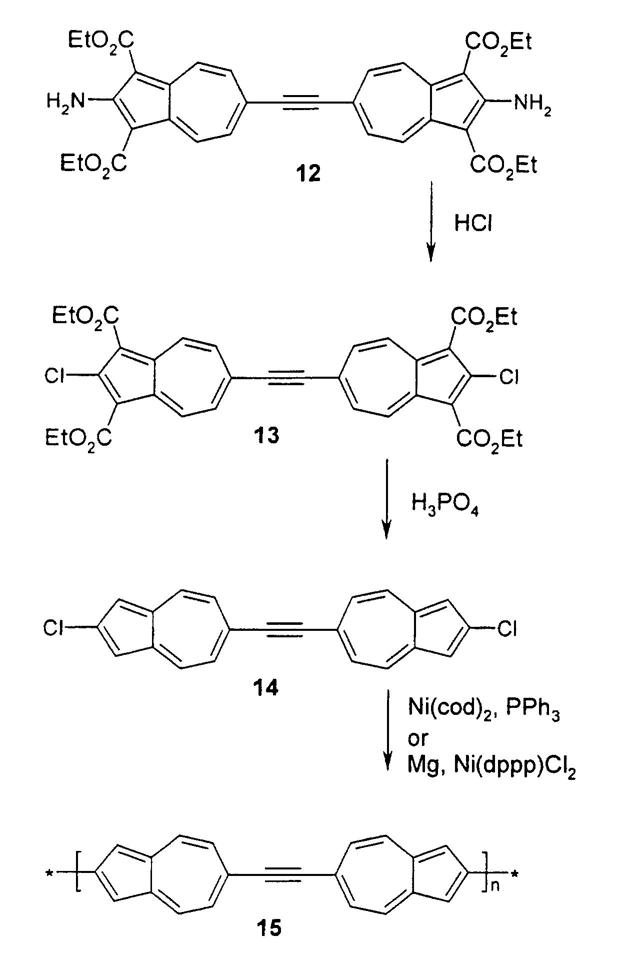

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICI, ICI₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCI, HNO₃, H₂SO₄, HClO₄, FSO₃H and ClSO₃H), transition metal compounds (e.g. FeCl₃, FeOCI, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃·6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns or tracts in electronic applications such as printed circuit boards and condensers.

The oligo- and polyazulenes of the present invention are useful as optical, electronic and semiconductor materials, in particular as charge transport materials in field effect transistors (FETs) e.g. as components of integrated circuitry, ID tags or TFT applications. Alternatively, they may be used in organic light emitting diodes (OLEDs) in electroluminescent display applications or as backlight of e.g. liquid crystal displays, as photovoltaics or sensor materials, for electrophotographic recording, and for other semiconductor applications.

The oligomers and polyazulenes according to the invention show advantageous solubility properties which allow production processes using solutions of these compounds. Thus films, including layers and coatings, may be generated by low cost production techniques e.g. spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semi-conductive material is arranged as a film between a gate-dielectric and a drain and a source electrode, are generally known e.g. from US 5,892,244, WO 00/79617, US 5,998,804, and from the references cited in the background and prior art chapter and listed below. Due to the advantages, like low cost production using the solubility properties of the compounds according to the invention and thus the processability of large surfaces, preferred applications of these FETs are such as integrated circuitry, TFT-displays and security applications.

In security applications, field effect transistors and other devices with semi-conductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the oligo- and polymers according to the invention may be used in organic light emitting devices or diodes (OLEDs), e.g. in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, *Synthetic Materials,* 111-112, 2000, 31-34, Alcala, *J. Appl. Phys*., 88, 2000, 7124-7128 and the literature cited therein.

According to another use, the inventive compounds, materials or films, especially those which show photoluminescent properties, may be employed as materials of light sources, e.g. of display devices such as described in EP 0 889 350 A1 or by C. Weder et al., *Science,* 279, 1998, 835-837.

## Claims

1. oligo- and polymers comprising at least two azulene-2,6-diyl groups or at least one [2,6']-bisazulene-6,2'-diyl, [2,2']-bisazulene-6,6'-diyl or [6,6']-bisazulene-2,2'-diyl group.

2. oligo- and polymers comprising identical or different recurring units of formula I
-[(A)ₐ-(B)_{b}-(C)_{c}]- I
wherein
A and C are independently of each other and independently in each occurrence -CX¹=CX²-, -C≡C-, or optionally substituted arylene or heteroarylene, or have one of the meanings of B,
X¹ and X² are independently of each other H, F, Cl or CN,
B is independently in each occurrence azulene-2,6-diyl, [2,6']-bisazulene-6,2'-diyl, [2,2']-bisazulene-6,6'-diyl, [6,6']-bisazulene-2,2'-diyl, all of which are substituted or unsubstituted, or a mirror image of one of these groups, and
a, b and c are independently of each other 0, 1 or 2, with a + b + c>0,
with the proviso that the oligo- and polymers comprise at least two azulene-2,6-diyl groups or at least one [2,6']-bisazulene-6,2'-diyl, [2,2']-bisazulene-6,6'-diyl or [6,6']-bisazulene-2,2'-diyl group.

3. oligo- and polymers according to claim 2, **characterized in that** they are selected of formula I1
R⁶-[(A)ₐ-(B)_{b}-(C)_{c}]ₙ- R² I1
wherein A, C, a, b and c are as defined in formula I,
B is independently in each occurrence a group selected of formulae IIa to IId or their mirror images 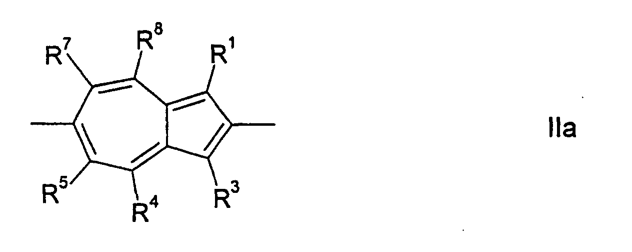 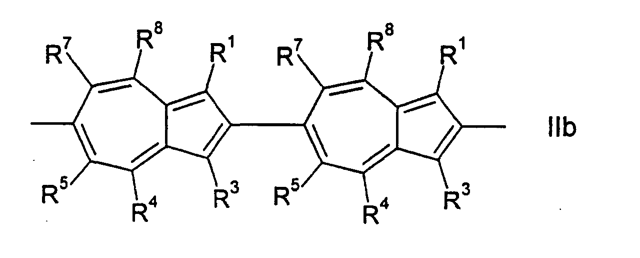 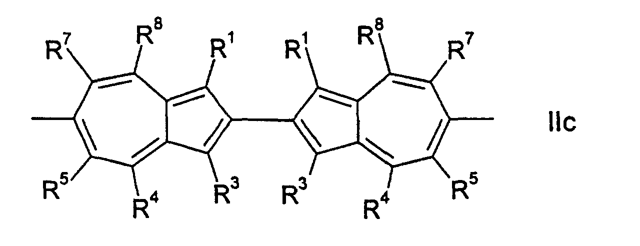 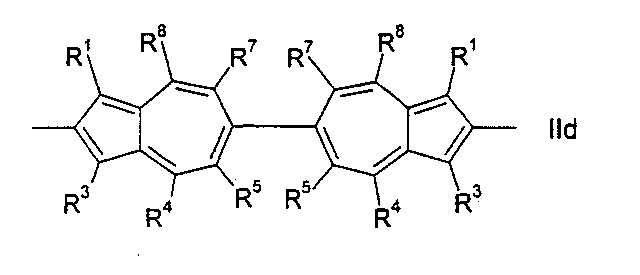
R¹ to R⁸ are independently of each other H, halogen or straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-,-NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl,
R⁰ and R⁰⁰ are independently of each other H or alkyl with 1 to 12 C-atoms,
n is an integer from 1 to 5000,
wherein the recurring units [(A)ₐ-(B)_{b}-(C)_{c}] can be identical or different.

4. oligo- and polymers according to at least one of claims 2 to 3, wherein n is an integer from 2 to 5000.

5. oligo- and polymers according to at least one of claims 2 to 4, wherein R¹, R³, R⁴, R⁵, R⁷ and R⁸ are selected from H, C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, and optionally substituted aryl or heteroaryl.

6. oligo- and polymers according to at least one of claims 2 to 5, wherein R² and R⁶ are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, and optionally substituted aryl or heteroaryl.

7. oligo- and polymers according to at least one of claims 2 to 6, **characterized in that** they are selected from the following formulae 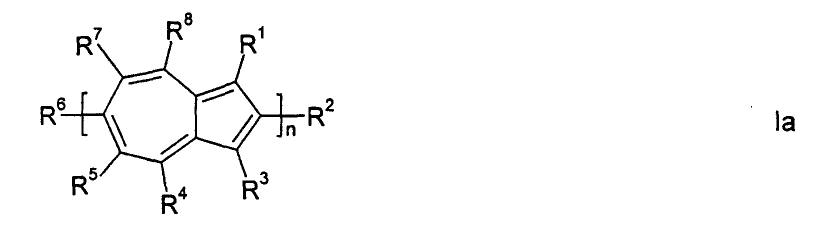 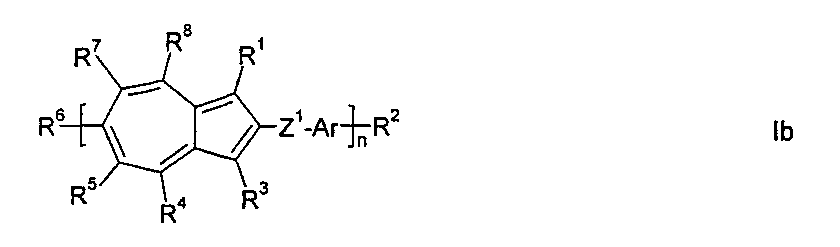 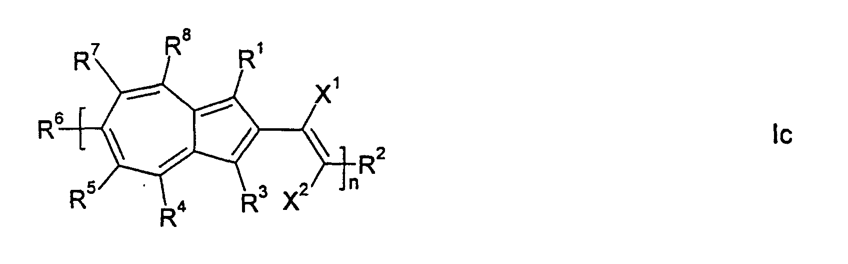 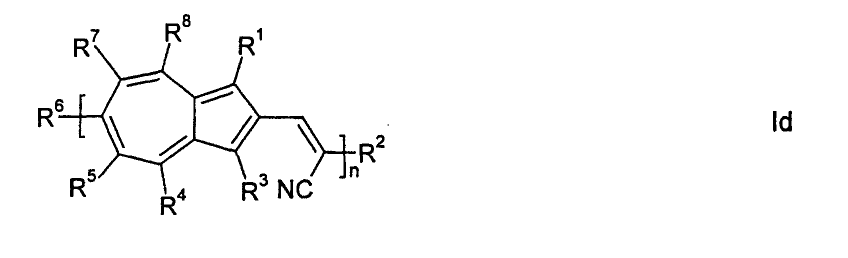 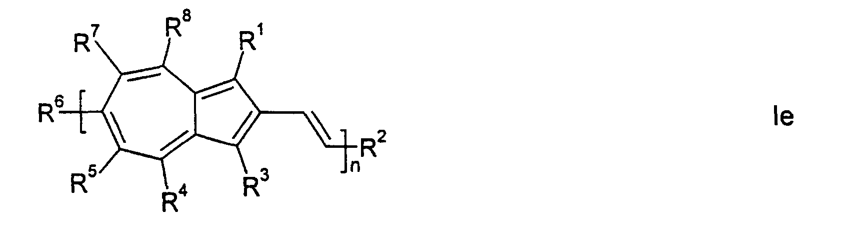 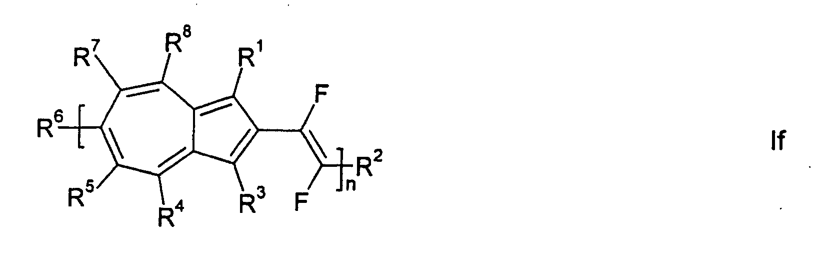 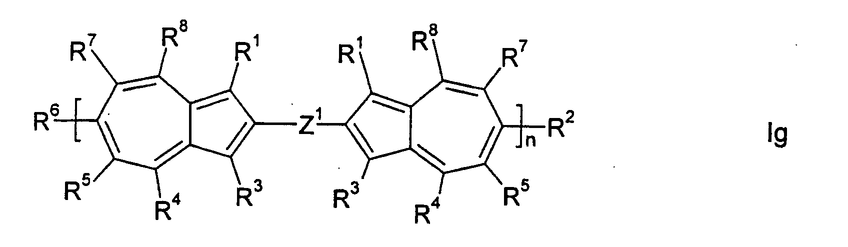 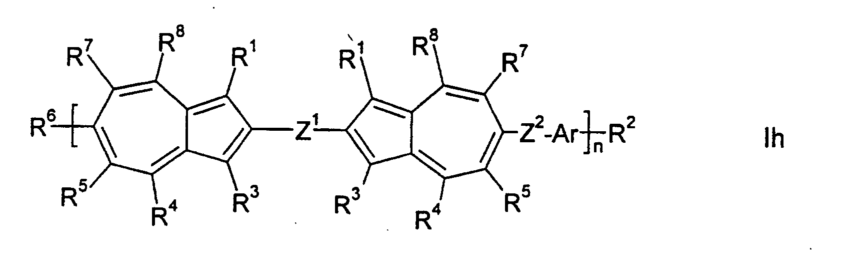 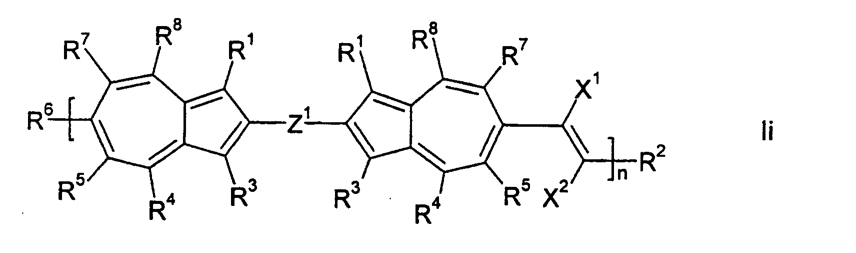 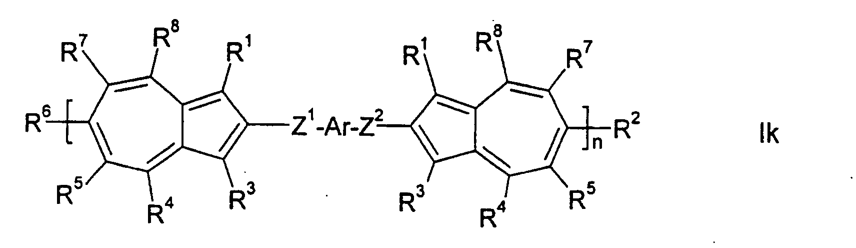 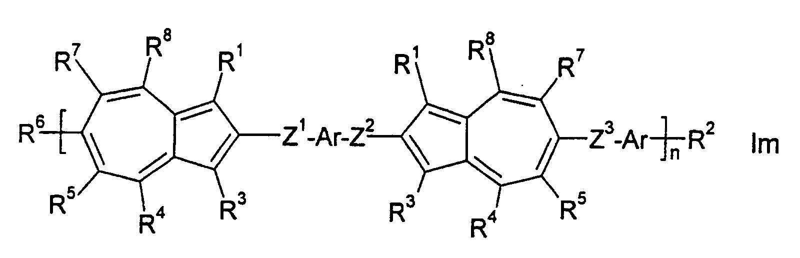 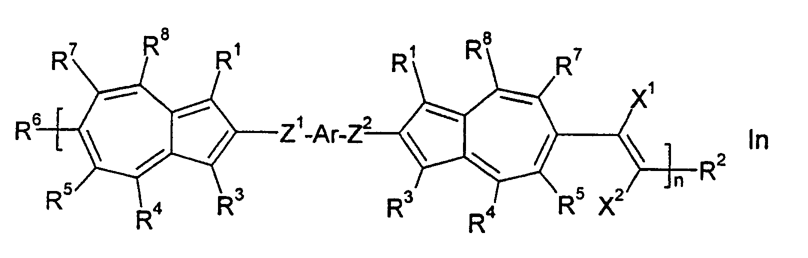 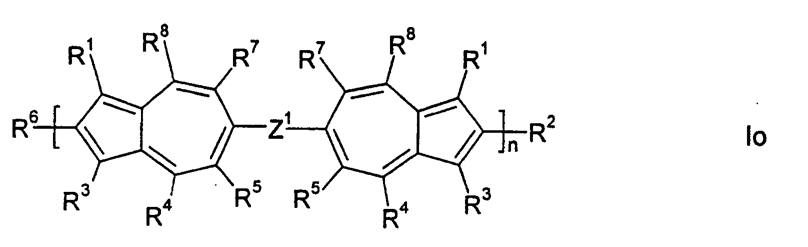 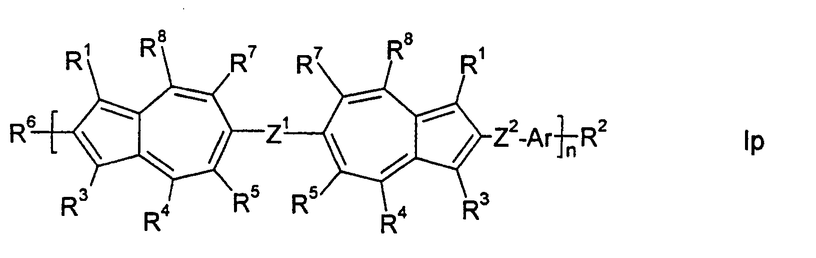 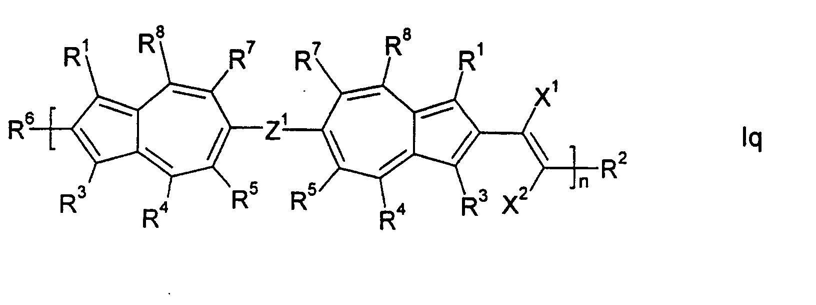 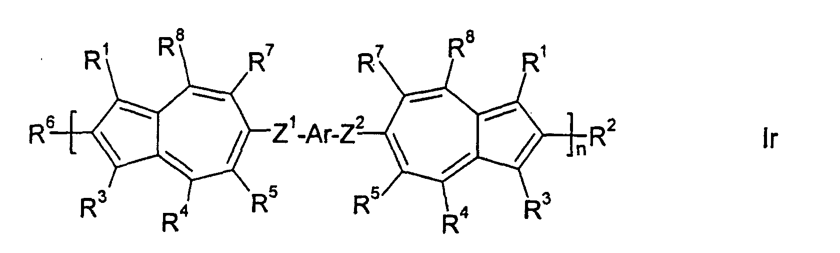 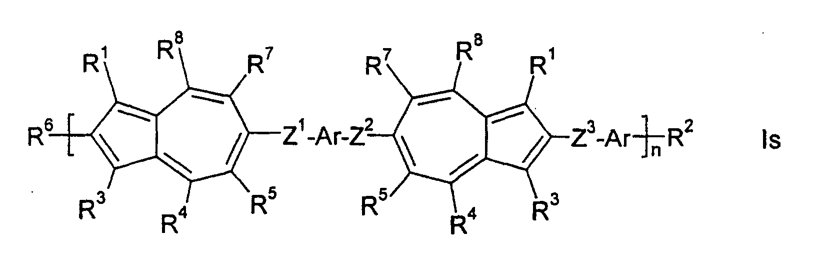 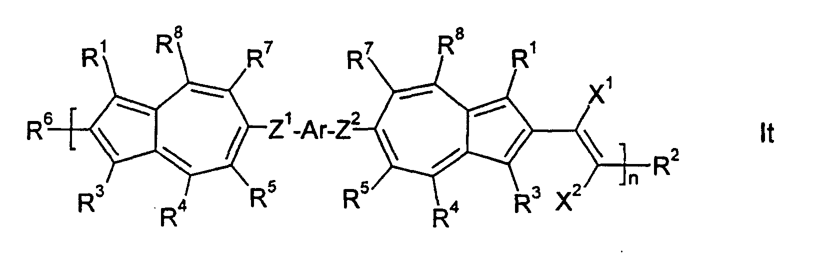 wherein R¹ to R⁸ and n have the meanings given in formula I1,
Ar is (ar)ₘ, with ar being arylene or heteroarylene and m being 1, 2 or 3,
Z¹, Z² and Z³ are independently of each other -CX¹=CX²-, C=C- or a single bond, and
X¹ and X² have the meanings given in formula I.

8. Use of the oligo- and polymers according to at least one of claims 1 to 7 as semiconductors or charge transport materials, in particular in optical, electrooptical or electronic devices, like for example components of integrated circuitry, field effect transistors (FET) for example as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors and for electrophotographic applications like electrophotographic recording.

9. Field effect transistor, for example as a component of integrated circuitry, as a thin film transistor in flat panel display applications, or in a Radio Frequency Identification (RFID) tag, comprising one or more oligo- or polymers according to at least one of claims 1 to 7.

10. Security marking or device comprising comprising one or more oligo- or polymers according to at least one of claims 1 to 7, or a FET or RFID tag according to claim 9.

11. oligo- and polymers according to at least one of claims 1 to 7, which are oxidatively or reductively doped to form conducting ionic species.

12. Charge injection layer, planarising layer, antistatic film or conducting substrate or pattern for electronic applications or flat panel displays, comprising one or more oligo- or polymers according to claim 11.

## Patentansprüche

1. Oligo- und Polymere, enthaltend mindestens zwei Azulen-2,6-diyl-Gruppen oder mindestens eine [2,6']-Bisazulen-6,2'-diyl-, [2,2']-Bisazulen-6,6'-diyl- oder [6,6']-Bisazulen-2,2'-diyl-Gruppe.

2. Oligo- und Polymere, enthaltend identische oder verschiedene wiederkehrende Einheiten der Formel I
-[(A)ₐ-(B)_{b}-(C)_{c}]- I,
worin:
A und C unabhängig voneinander und unabhängig bei jedem Vorkommen -CX¹=CX²-, -C≡C- oder gegebenenfalls substituiertes Arylen oder Heteroarylen sind, oder eine der Bedeutungen von B haben,
X¹ und X² unabhängig voneinander H, F, Cl oder CN sind,
B unabhängig bei jedem Vorkommen Azulen-2,6-diyl, [2,6']-Bisazulen-6,2'-diyl, [2,2']-Bisazulen-6,6'-diyl, [6,6']-Bisazulen-2,2'-diyl ist, die jeweils substituiert oder unsubstituiert sind, oder ein Spiegelbild von einer dieser Gruppen und
a, b und c unabhängig voneinander 0, 1 oder 2 sind, wobei a + b + c > 0 gilt,
mit der Maßgabe, dass die Oligo- und Polymere mindestens zwei Azulen-2,6-diyl-Gruppen oder mindestens eine [2,6']-Bisazulen-6,2'-diyl-, [2,2']-Bisazulen-6,6'-diyl- oder [6,6']-Bisazulen-2,2'-diyl-Gruppe enthalten.

3. Oligo- und Polymere nach Anspruch 2, **dadurch gekennzeichnet, dass** sie ausgewählt sind aus Formel I1
R⁶-[(A)ₐ-(B)_{b}-(C)_{c}]ₙ- R² I1,
worin A, C, a, b und c die in Formel I angegebene Bedeutung haben,
B unabhängig bei jedem Vorkommen eine Gruppe ist, ausgewählt aus den Formeln IIa bis IId oder ihren Spiegelbildern 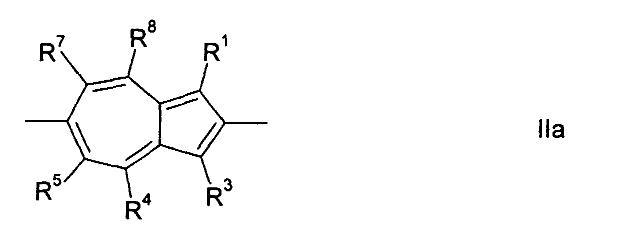 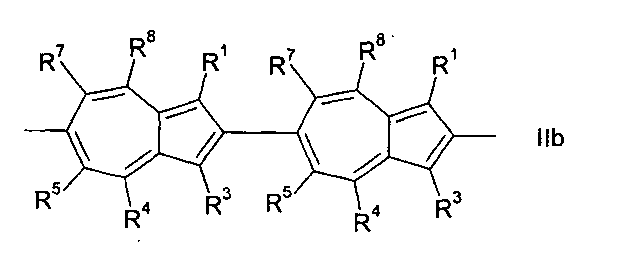 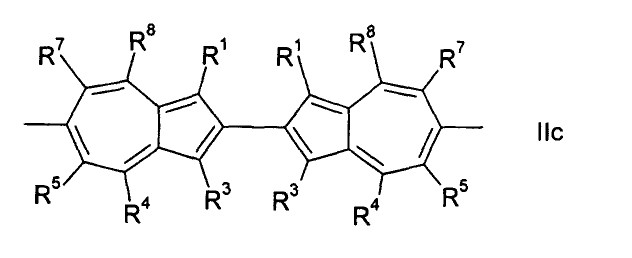 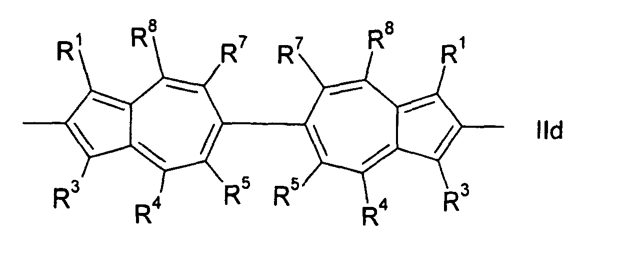
R¹ bis R⁸ unabhängig voneinander H, Halogen oder geradkettiges, verzweigtes oder cyclisches Alkyl mit 1 bis 20 C-Atomen, das unsubstituiert, ein- oder mehrfach durch F, Cl, Br, I oder CN substituiert ist, wobei gegebenenfalls eine oder mehrere nicht benachbarte CH₂-Gruppen jeweils unabhängig voneinander durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- so ersetzt sind, dass O und/oder S-Atome nicht direkt miteinander verknüpft sind, oder gegebenenfalls substituiertes Aryl oder Heteroaryl sind,
R⁰ und R⁰⁰ unabhängig voneinander H oder Alkyl mit 1 bis 12 C-Atomen sind,
n eine ganze Zahl von 1 bis 5000 ist,
wobei die Wiederholungseinheiten [(A)ₐ-(B)_{b}-(C)_{c}] identisch oder verschieden sein können.

4. Oligo- und Polymere nach mindestens einem der Ansprüche 2 bis 3, wobei n eine ganze Zahl von 2 bis 5000 ist.

5. Oligo- und Polymere nach mindestens einem der Ansprüche 2 bis 4, worin R¹, R³, R⁴, R⁵, R⁷ und R⁸ ausgewählt sind aus H, C₁-C₂₀-alkyl, das gegebenenfalls substituiert ist mit einem oder mehreren Fluoratomen, C₁-C₂₀-Alkenyl, C₁-C₂₀-Alkinyl, C₁-C₂₀-Alkoxy, C₁-C₂₀-Thioether, C₁-C₂₀-Silyl, C₁-C₂₀-Ester, C₁-C₂₀-Amino, C₁-C₂₀-Fluoralkyl, und gegebenenfalls substituiertem Aryl oder Heteroaryl.

6. Oligo- und Polymere nach mindestens einem der Ansprüche 2 bis 5, worin R² und R⁶ ausgewählt sind aus C₁-C₂₀-Alkyl, das gegebenenfalls substituiert ist mit einem oder mehreren Fluoratomen, C₁-C₂₀-Alkenyl, C₁-C₂₀-Alkinyl, C₁-C₂₀-Alkoxy, C₁-C₂₀-Thioether, C₁-C₂₀-Silyl, C₁-C₂₀-Ester, C₁-C₂₀-Amino, C₁-C₂₀-Fluoralkyl, und gegebenenfalls substituiertem Aryl oder Heteroaryl.

7. Oligo- und Polymere nach mindestens einem der Ansprüche 2 to 6, **dadurch gekennzeichnet, dass** sie aus den folgenden Formeln ausgewählt sind: 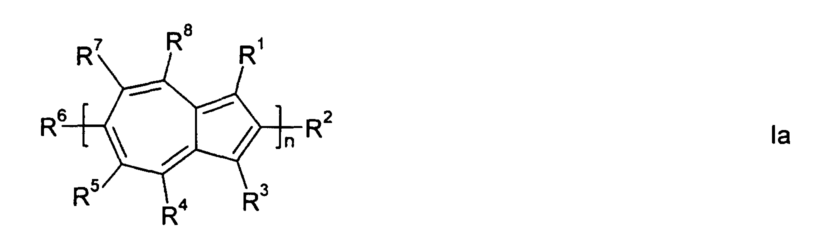 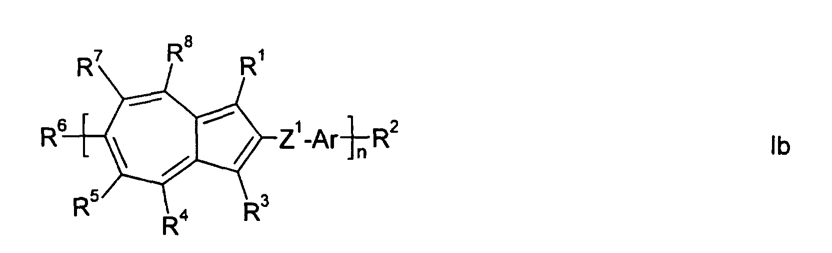 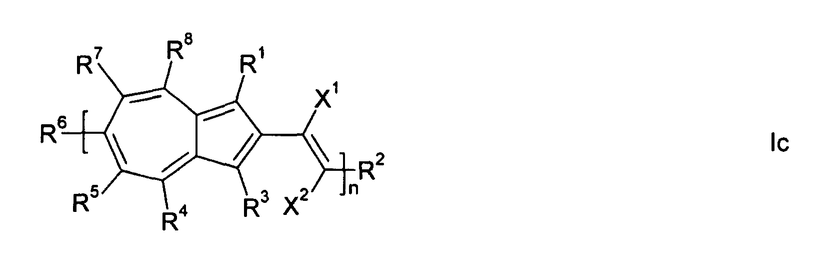 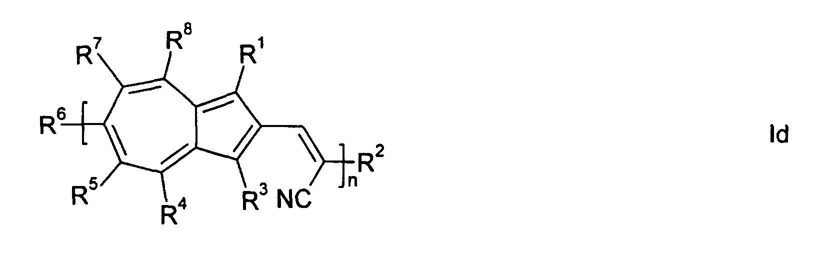 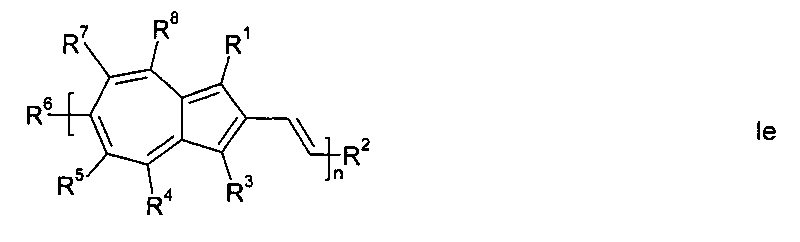 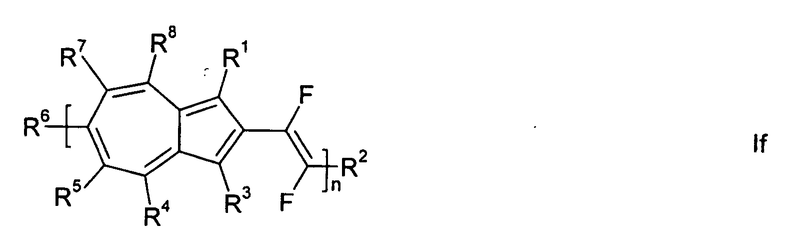 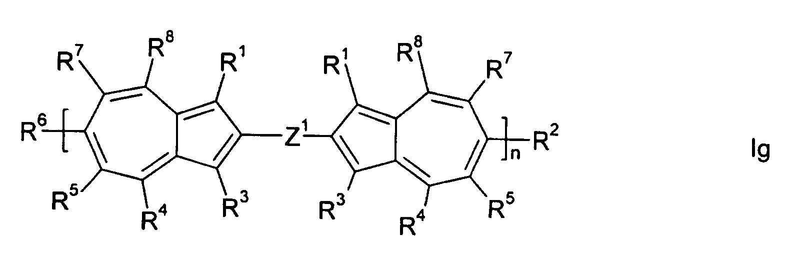 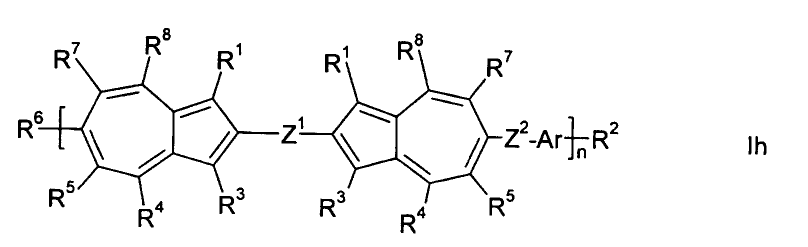 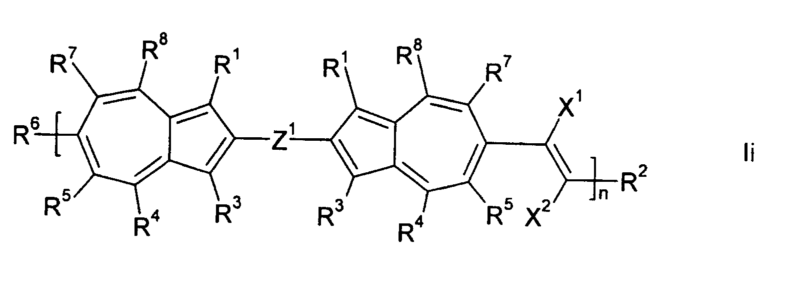 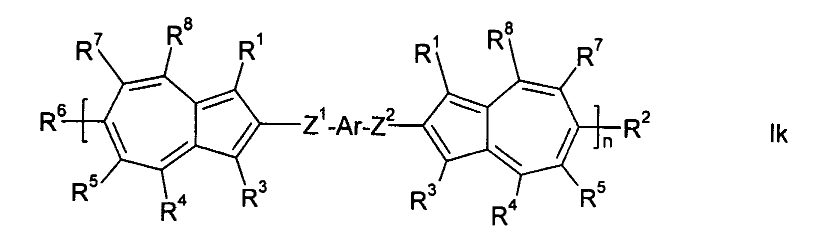 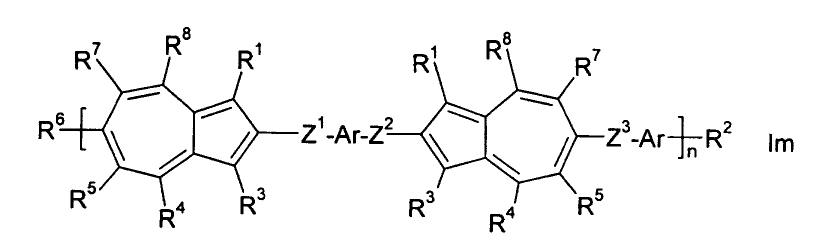 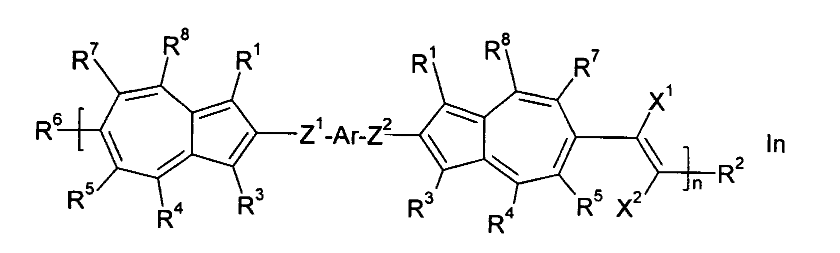 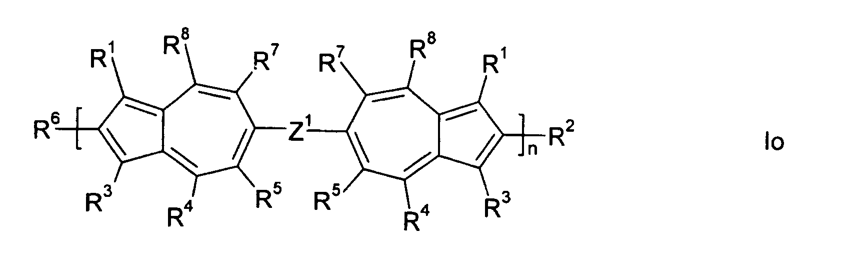 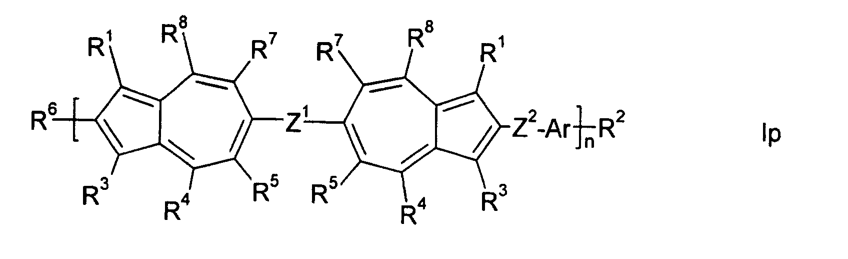 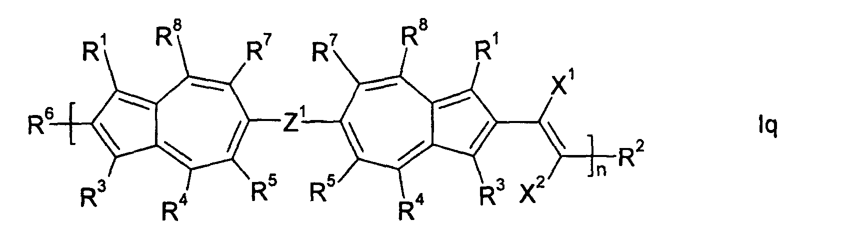 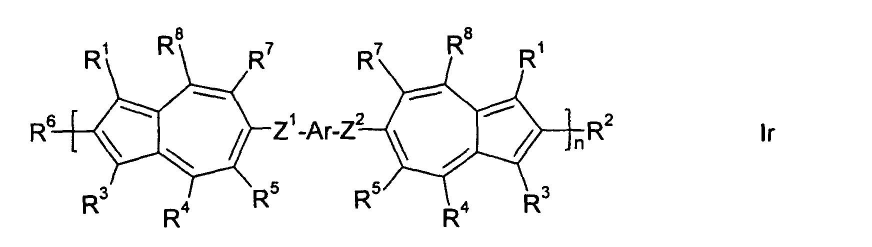 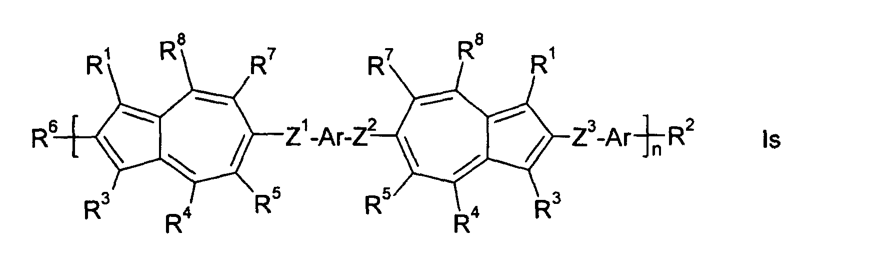 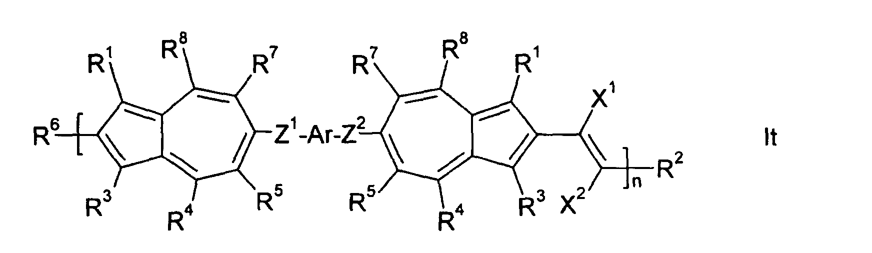 worin R¹ bis R⁸ und n die in Formel 11 angegebenen Bedeutungen haben,
Ar (ar)ₘ ist, wobei Ar Arylen oder Heteroarylen ist und m 1, 2 oder 3 ist,
Z¹, Z² und Z³ unabhängig voneinander -CX¹=CX²-, C≡C- oder eine Bindung sind, und
X¹ und X² die in Formel I angegebenen Bedeutungen haben.

8. Verwendung der Oligo- und Polymere nach mindestens einem der Ansprüche 1 bis 7 als Halbleiter oder Ladungstransportmaterialien, insbesondere bei optischen, elektrooptischen oder elektronischen Vorrichtungen, wie z.B. Komponenten für integrierte Schaltungen, Feldeffekttransistoren (FET), beispielsweise als Dünnfilm-Transistoren in Flachbildschirm-Anwendungen oder für Radiofrequenzidentifikations-(RFID)-Tags oder in Halbleiterbauteilen für Anwendungen in organische Leuchtdioden (OLED), wie Elektrolumineszenzanzeigen oder Hintergrundbeleuchtung von z.B. Flüssigkristallanzeigen, für Photovoltaik- oder Sensor-Vorrichtungen, als Elektrodenmaterialien in Batterien, als Photoleiter und für elektrophotographische Anwendungen, wie elektrophotographische Aufzeichnung.

9. Feldeffekttransistor, beispielsweise als Komponente für integrierte Schaltungen, als Dünnfilm-Transistor in Flachbildschirmanwendungen oder bei Radiofrequenzidentifikations-(RFID)-Tags, enthaltend ein oder mehrere Oligo- oder Polymere nach mindestens einem der Ansprüche 1 bis 7.

10. Sicherheits-Markierung oder -Vorrichtung enthaltend eine oder mehrere Oligo- oder Polymere nach mindestens einem der Ansprüche 1 bis 7 oder ein FET oder ein RFID-Tag nach Anspruch 9.

11. Oligo- und Polymere nach mindestens einem der Ansprüche 1 bis 7, die oxidativ oder reduktiv dotiert sind, so dass sie leitende lonenspezies bilden.

12. Ladungsinjektionsschicht, Planarisierungsschicht, Antistatikfolie oder leitendes Substrat oder Muster für Elektronikanwendungen oder Flachbildschirme, enthaltend ein oder mehrere Oligo- oder Polymere nach Anspruch 11.

## Revendications

1. Oligomères et polymères comprenant au moins deux groupes azulène-2,6-diyle ou au moins un groupe [2,6']-bisazulène-6,2'-diyle, [2,2']-bisazulène-6,6'-diyle ou [6,6']-bisazulène-2,2'-diyle.

2. Oligomères et polymères comprenant, des unités récurrentes identiques ou différentes de la formule I
-[(A)ₐ-(B)_{b}-(C)_{c}]- I
dans laquelle
A et C sont indépendamment l'un de l'autre et de manière indépendante au niveau de chaque occurrence -CX¹=CX²-, -C≡C-, ou sont arylène ou hétéroarylène en option substitué, ou présentent l'une des significations de B,
X¹ et X² sont indépendamment l'un de l'autre H, F, Cl ou CN,
B est de manière indépendante au niveau de chaque occurrence azulène-2,6-diyle, [2,6']-bisazulène-6,2'-diyle, [2,2']-bisazulène-6,6'-diyle, [6,6']-bisazulène-2,2'-diyle, dont tous peuvent être substitués ou non substitués, ou une image miroir de l'un de ces groupes, et
a, b et c sont indépendamment les uns des autres 0, 1 ou 2, moyennant a + b + c > 0,
étant entendu que les oligomères et polymères comprennent au moins deux groupes azulène-2,6-diyle ou au moins un groupe [2,6']-bisazulène-6,2'-diyle, [2,2']-bisazulène-6,6'-diyle ou [6,6']-bisazulène-2,2'-diyle.

3. Oligomères et polymères selon la revendication 2, **caractérisés en ce qu'**ils sont choisis à partir de la formule I1
R⁶-[(A)ₐ-(B)_{b}-(C)_{c}]ₙ- R² I1
dans laquelle A, C, a, b et c sont comme défini selon la formule I,
B est de manière indépendante au niveau de chaque occurrence un groupe choisi à partir des formules IIa à IId ou leurs images miroirs 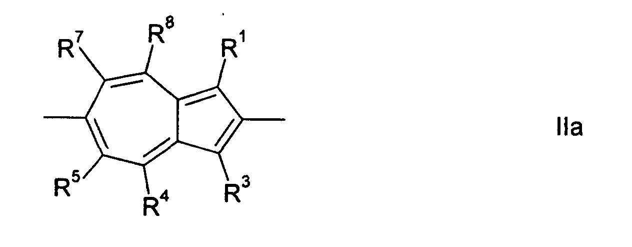 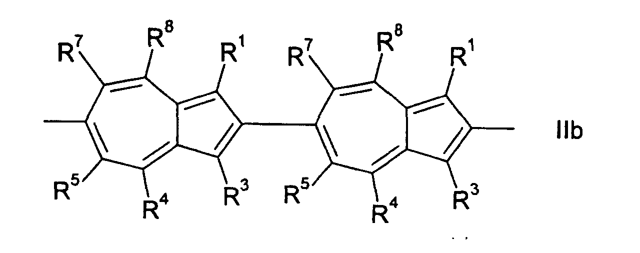 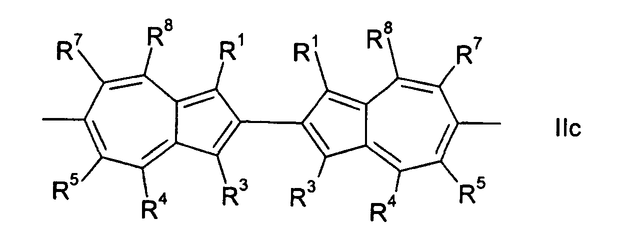 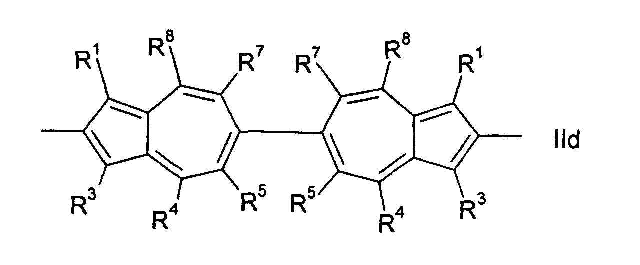
R¹ à R⁸ sont indépendamment les uns des autres H, halogène ou alkyle en chaîne droite, ramifié ou cyclique avec de 1 à 20 atomes de C, qui est non substitué, mono ou polysubstitué par F, Cl, Br, I ou CN, et où un ou plusieurs groupes CH₂ non adjacents sont en option remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas liés directement les uns aux autres, ou sont en option aryle ou hétéroaryle substitué,
R⁰ et R⁰⁰ sont indépendamment l'un de l'autre H ou alkyle avec de 1 à 12 atomes de C,
n est un entier de 1 à 5000,
où les unités récurrentes [(A)ₐ-(B)_{b}-(C)_{c}] peuvent être identiques ou différentes.

4. Oligomères et polymères selon au moins l'une des revendications 2 et 3, dans lesquels n est un entier de 2 à 5000.

5. Oligomères et polymères selon au moins l'une des revendications 2 à 4, dans lesquels R¹, R³, R⁴, R⁵, R⁷ et R⁸ sont choisis à partir de H, C₁-C₂₀-alkyle qui est en option substitué par un ou plusieurs atomes de fluor, C₁-C₂₀-alkényle, C₁-C₂₀-alkynyle, C₁-C₂₀-alkoxy, C₁-C₂₀-thioéther, C₁-C₂₀-silyle, C₁-C₂₀-ester, C₁-C₂₀-amino, C₁-C₂₀-fluoro-alkyle, et en option aryle ou hétéroaryle substitué.

6. Oligomères et polymères selon au moins l'une des revendications 2 à 5, dans lesquels R² et R⁶ sont choisis à partir de C₁-C₂₀-alkyle qui est en option substitué par un ou plusieurs atomes de fluor, C₁-C₂₀-alkényle, C₁-C₂₀-alkynyle, C₁-C₂₀-alkoxy, C₁-C₂₀-thioéther, C₁-C₂₀-silyle, C₁-C₂₀-ester, C₁-C₂₀-amino, C₁-C₂₀-fluoroalkyle, et en option aryle ou hétéroaryle substitué.

7. Oligomères et polymères selon au moins l'une des revendications 2 à 6, **caractérisés en ce qu'**ils sont choisis à partir des formules qui suivent 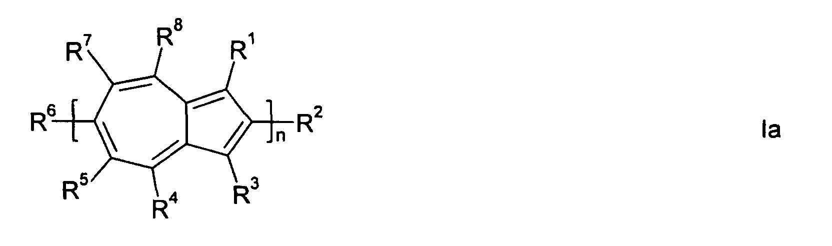 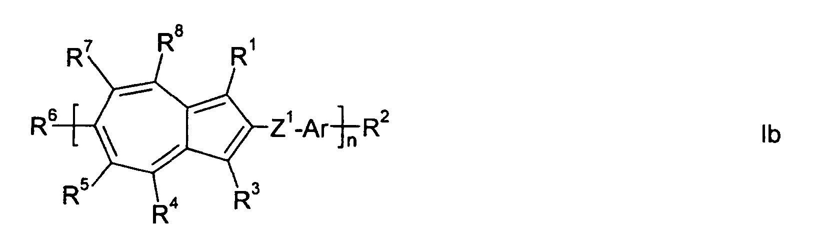 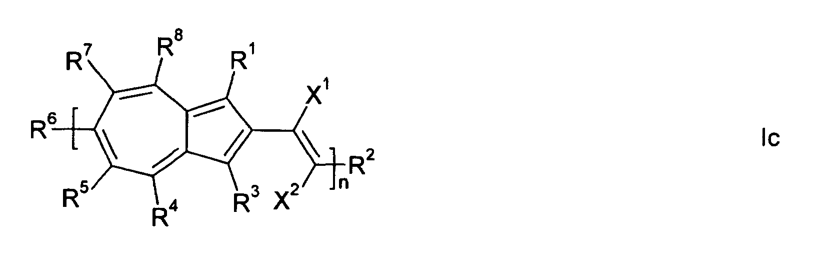 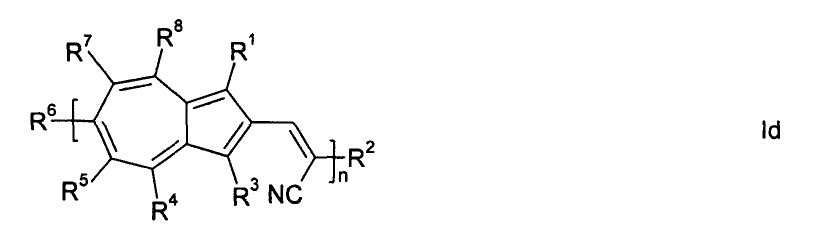 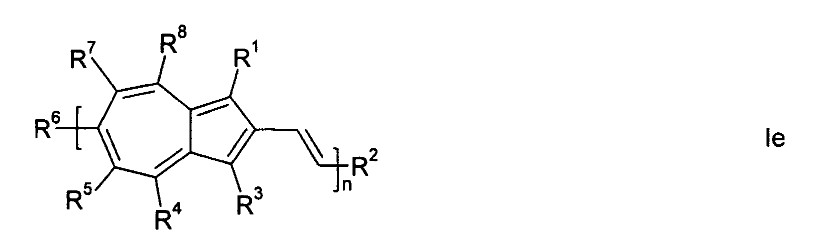 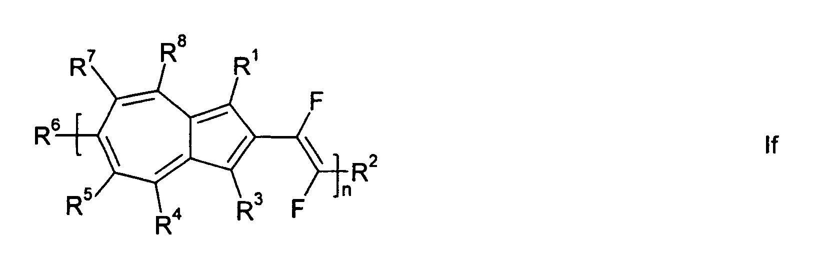 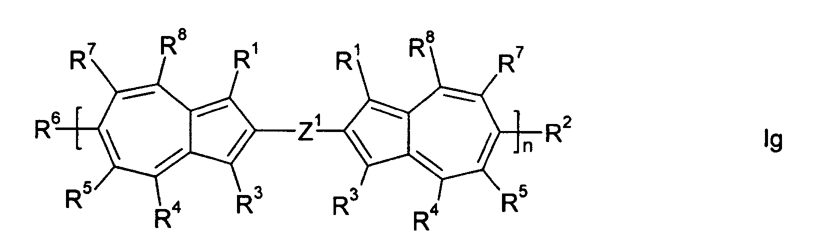 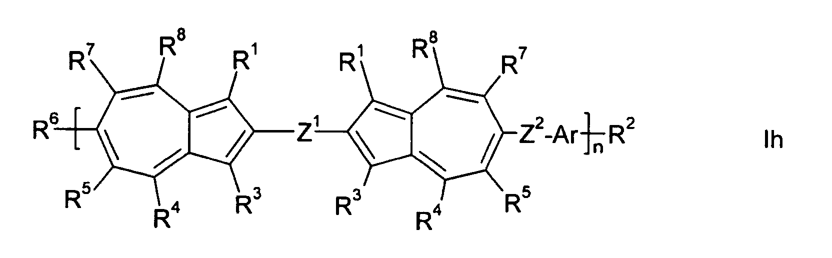 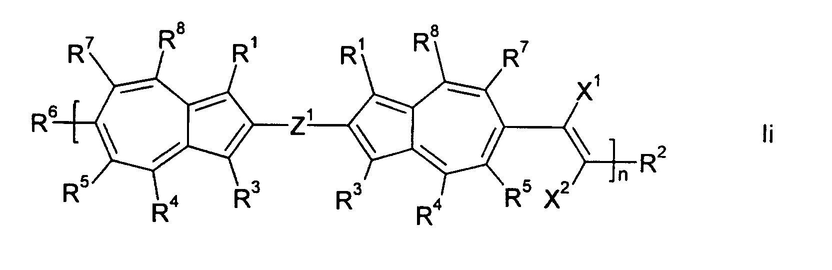 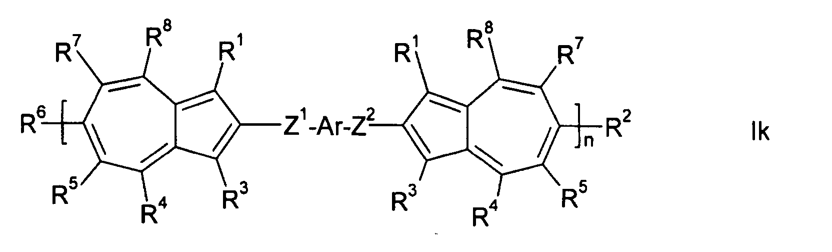 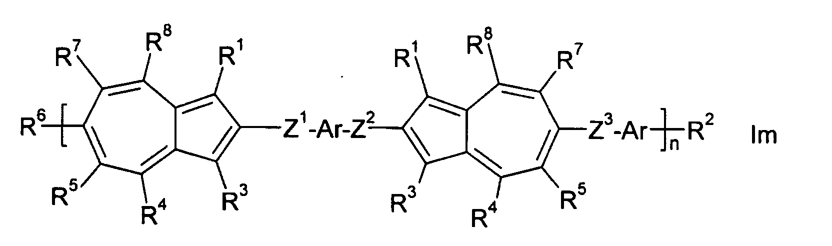 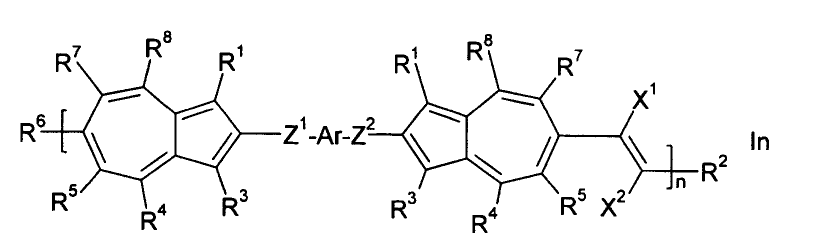 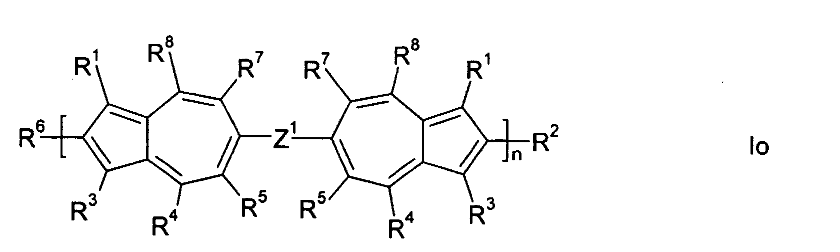 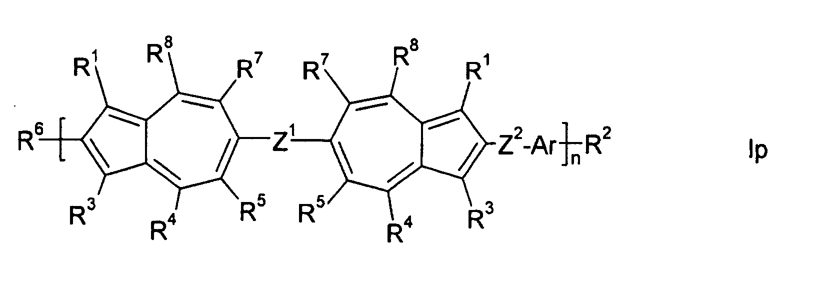 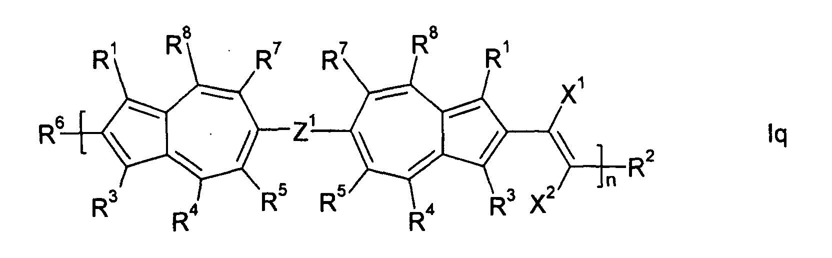 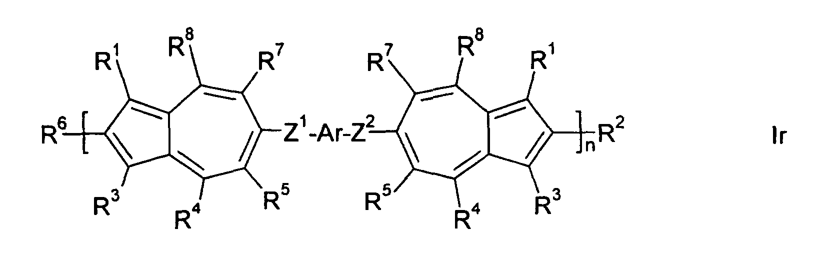 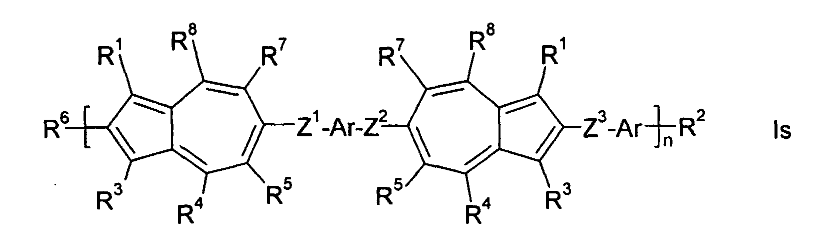 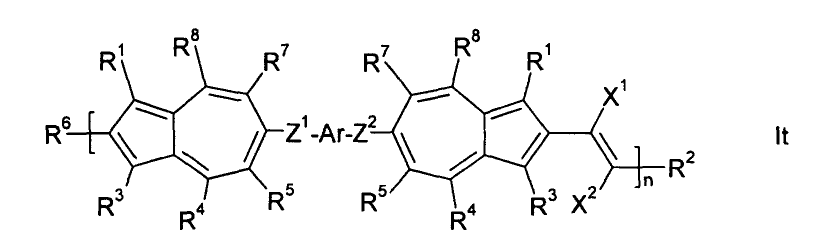 dans lesquelles R¹ à R⁸ et n présentent les significations données selon la formule I1,
Ar est (ar)ₘ, ar étant arylène ou hétéroarylène et m étant 1, 2 ou 3,
Z¹, Z² et Z³ sont indépendamment les uns des autres -CX¹=CX²-, C≡C- ou une liaison simple, et
X¹ et X² présentent les significations données selon la formule I.

8. Utilisation des oligomères et polymères selon au moins l'une des revendications 1 à 7 en tant que semiconducteurs ou matériaux de transport de charges, en particulier dans des dispositifs optiques, électro-optiques ou électroniques, tels que par exemple des composants de circuit intégré, des transistors à effet de champ (FET) par exemple en tant que transistors à film mince dans des applications d'affichage à écran plat ou pour des étiquettes d'Identification Radio Fréquence (RFID), ou dans des composants semiconducteurs pour des applications de diodes émettrices de lumière organiques (OLED) telles que des affichages électroluminescents ou des éclairages arrière de par exemple des affichages à cristaux liquides, pour des dispositifs photovoltaïques ou des dispositifs de capteur, en tant que matériaux d'électrode dans des accumulateurs, en tant que photoconducteurs et pour des applications électrophotographiques telles qu'un enregistrement électrophotographique.

9. Transistor à effet de champ, par exemple en tant que composant de circuit intégré, en tant que transistor à film mince dans des applications d'affichage à écran plat, ou dans une étiquette d'identification Radio Fréquence (RFID), comprenant un ou plusieurs oligomères ou polymères selon au moins l'une des revendications 1 à 7.

10. Marquage ou dispositif de sécurité comprenant un ou plusieurs oligomères ou polymères selon au moins l'une des revendications 1 à 7, ou un FET ou une étiquette RFID selon la revendication 9.

11. Oligomères et polymères selon au moins l'une des revendications 1 à 7, qui sont dopés de façon oxydante ou réductrice afin de former des espèces ioniques conductrices.

12. Couche d'injection de charges, couche de planarisation, film antistatique ou substrat ou motif conducteur pour des applications électroniques ou des affichages à écran plat, comprenant un ou plusieurs oligomères ou polymères selon la revendication 11.
